# EUROPEAN PATENT SPECIFICATION

(11) **EP 1 069 119 B1**
(45) Date of publication and mention of the grant of the patent: **15.09.2004**
(21) Application number: 99305565.6
(22) Date of filing: 14.07.1999
(51) Int. Cl.: C07D 285/12, H01M 10/40

(54) **2,5-dimercapto-1,3,4-thiadiazole dilithium salt and its dihydrate and their manufacture**
2,5-Dimercapto-1,3,4-thiadiazol-Dilithiumsalz und sein Dihydrat sowie ihre Herstellung
Sel disodium de 2,5-dimercapto-1,3,4-thiadiazole et son dihydrate ainsi que leur preparation

(43) Date of publication of application: 17.01.2001
(73) Proprietor: Toyo Kasei Kogyo Company Limited, Osaka 530-0004 (JP)
(72) Inventor: Yamaguchi, Satoshi, Research Laboratory, Takasago City, Hyogo Prefecture (JP); Nakagawa, Satoshi, Research Laboratory, Takasago City, Hyogo Prefecture (JP); Mitsui, Sunao, Research Laboratory, Takasago City, Hyogo Prefecture (JP); Ohno, Susumu, Research Laboratory, Takasago City, Hyogo Prefecture (JP)
(74) Representative: Wilkinson, Stephen John

(56) References cited:
- EP-A- 0 497 308
- N. OYAMA ET AL.: NATURE, vol. 373, 16 February 1995 (1995-02-16), pages 598-600, XP002128025
- PATENT ABSTRACTS OF JAPAN vol. 17, no. 57 (E-1315), 4 February 1993 (1993-02-04) & JP 04 267074 A (MATSUSHITA ELECTRIC IND CO LTD), 22 September 1992 (1992-09-22)
- PATENT ABSTRACTS OF JAPAN vol. 17, no. 57 (E-1315), 4 February 1993 (1993-02-04) & JP 04 267073 A (MATSUSHITA ELECTRIC IND CO LTD), 22 September 1992 (1992-09-22)
- PATENT ABSTRACTS OF JAPAN vol. 17, no. 514 (E-1433), 16 September 1993 (1993-09-16) & JP 05 135799 A (MATSUSHITA ELECTRIC IND CO LTD), 1 June 1993 (1993-06-01)
- PATENT ABSTRACTS OF JAPAN vol. 17, no. 514 (E-1433), 16 September 1993 (1993-09-16) & JP 05 135800 A (MATSUSHITA ELECTRIC IND CO LTD), 1 June 1993 (1993-06-01)
- PATENT ABSTRACTS OF JAPAN vol. 18, no. 116 (E-1515), 24 February 1994 (1994-02-24) & JP 05 314964 A (MATSUSHITA ELECTRIC IND CO LTD), 26 November 1993 (1993-11-26)

## Description

This invention relates to a dilithium salt of 2,5-dimercapto-1,3,4-thiadiazole (DMc T dilithium salt) and its new dihydrate and their manufacture. DMc T dilithium salt dihydrate, new compounds manufactured by this invention. DMc T dilithium salt and its dihydrate are industrially very useful substances which are used widely, for example, as a photosensitive material for photos, a dye, an inhibitor, a surface-treating agent for metals, a raw material for additives to lubricating oils, a monomer for high refractive index plastics, an electrode material for lithium batteries, and a starting material or intermediate for medicines and agricultural chemicals.
(1) DMc T dilithium salt (abbreviated to DMc T-2 L i hereinafter) is very hygroscopic and deliquescent and its isolation is very difficult; therefore nothing has been reported on its properties and its handling has been hard. In addition, neither has the existence of its dihydrate been clearly reported nor it has been even suggested. Besides it is known that thiol compounds are easy to oxidise and that addition of alkali to them further facilitates their oxidation. This is true also of DMc T-2 L i; DMc T-2 L i is easily oxidised by air. This has raised a problem in trying isolation of DMc T-2 L i; the crystallisation doesn't go well when the compound's purity is low, and yet the application of a purification process in the compound makes its purity lower because of oxidation.
   AND
(2) The need for size and weight reduction of portable equipment and the consideration on the environment and energy issues have promoted very much the research and development of high energy density secondary batteries for years.

Particulary secondary lithium batteries have been developed remarkably. While at present the positive electrode material is mainly a metal oxide such as lithium cobaltate, etc., research on the use of organic compounds as a positive electrode material has been attracting attention on the grounds that such an electrode may achieve higher energy density than that of today's batteries using metal oxides. Of the organic compounds thiols are the most promisinng, and attempts to make use of the reversibility of thiols' oxidation and reduction reaction for the second battery have been made. One of those attempts was made by Koyama et al., who reported on the use of DMc T as a positive electrode material (Nature, vol. 373, No. 6515, p. 598-600, 1995); the battery in this reprot achieved high energy density by using as positive electrode a composite electrode made of conductive polyaniline and the thiol compound. In another example, i. e., in tokukaihei (Japanese PatentApplication Publication No.) 4-26704, Ueno et al. reported the use of lithium thiolate as a positive electrode material, and metallic aluminum or its alloy together with a carbon material as a negative electrode. Besides the current-voltage characteristic is lowered when an aluminum alloy containing 30 atom% lithium is used as a negative electrode; form this result, there will be a problem of battery performance degradation if metallic lithium is used as a negative electrode.

Further
(3)The inventors of the present invention developed various polythiol compounds and studied batteries using those compounds as a positive electrode material, but could not obtain a favorable battery because of the following problems:
   ① bad cycle characteristics
   ② odor of the compound used
   ③ generation of hydrogen gas during the initial charging and discharging
   ④ low heat stability of the compound used
   ⑤ in the case of a liquid polythiol compound, vaporization of the polythiol in the positive electrode-drying process

The use of the lithium salts of those polythiols as a positive electrode-active material and metallic lithium as a negative electrode-active material resulted in further lowering the cycle characteristics and also posed a problem of hydrogen gas generation during the initial charging and discharging.

### [Problems to Be Solved by the Invention]

The objective of this invention is to solve the problems listed above and provide the compounds of this invention and the method of their manufacture, the compounds of this invention having as mentioned above a wide range of uses as a photosensitive material for photos, a dye, an inhibitor, a surface-treating agent for metals, a raw material for additives to lubricating oils, a monomer for high refractive index plastics, an electrode material for lithium batteries, and a starting material or intermediate for medicines and agricultural chemicals.

### [Means for Solving the Problems]

The inventors worked very hard to accomplish the objective mentioned above and completed this invention. The first invention is new DMc T dilithium salt dihydrate represented the formula (1) below: 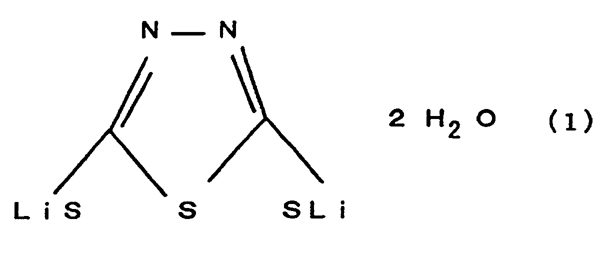 The second invention relates to a method for the manufacture of new DMc T dilithium salt dihydrade in which DMc T is allowed to react with a lithiation agent in a solvent at 0-120 °C, the mole ratio of lithium in the lithiation agent to DMc T being adjusted to 1.8-3.0.
The third invention is a method for the manufacture of DMc T dilithium salt represented by the formula (2) in which DMc T dilithium salt is obtaied by heating and dehydrating DMc T dilithium salt dihydrate represented by the formula(1) above at 200°C or higher and drying the product.

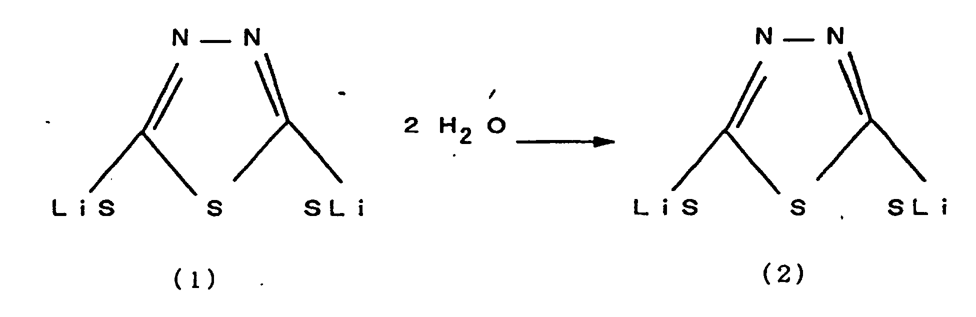

It has been found that DMc T-2Li dihydrade of this invention is of high quality when in the reaction of DM c T with a lithiation agent the mole ratio of lithium in the lithiation agent to DMc T is adjusted to 2. It has been also found in this invention that DMc T-2 L i dihydrate is less hygroscopic and less diliquescent than anhydrous DMc T-2 L i so the dihydrate has an advavtage over the anhydrous salt in isolability and handleability. The reaction of DMc T with a lithiation agent is carried out with the mole ration of lithium in the lithiation agent to DMc T of 2. When the mole ratio is greater than 2, DMc T-2 L i dihydrate is obtained but its purity is lowered because of the remaining lithiation agent. When the mole ratio is less than 2, the thiol groups remains, resulting in greater formability of the disulfide by-products by the reason mentioned above and less stability of the product compound. Thus the mole ration of lithium in the lithiation agent to DM c T should be 1.8-3.0 or more preferably 1. 9-2. 5.
Any lithiation agent can be used so far as it is one commonly used for that purpose; lithium hydroxide, lithium carbonate, etc. are preferable because they are very available and less expensive; particulary lithium hydroxide is more preferable because its high purity article is on the market. As for a solvent for the reaction, one which can dissolve DMc T-2 L i is preferable; the following can be used; water and alcohls such as methanol, ethanol, etc., polar aprotic solvents like dimethlformamide (DMF), and solvents such as tetrahydrofuran (THF), dioxane,etc.The following solvents can also be used in this synthesis if alcohl or water and alcohol are added: ethers such as diethyl ether, etc., aromatic compounds such as benzene toluene, etc., aliphatic compounds such as n-hexane, etc., and ketones such as acetone, methyl ethyl ketone,etc. The reaction temperature should be 0-120°C because of decomposition of DMc T at higher temperatures, and the more preferable temperature is 10 - 100°C. The appropriate reaction time is 1 minute - 10 hours, or more preferably 5 minutes- I hours. Besides DM c T is known to decompose at its melting point, i.e., at about 172°C; however, the decomposition temperature is much lowered when impurities are contained. The inventors' study has confirmed that the decomposition temperature is about 30°C lowered to 140°C when several percent of impurities from oxidation of DMc T are contained. Therefore DMc T is not a favorable compound if reactions or tretments at high temperatures are required.
The inventors have confirmed that, by changing the polythiol compound into its lithium salt and then by isolating the salt and drying to make it anhydrous, the hydrogen generation can be avoided completely and the odor emission in the process of the positive electrode fabrication,which was a problem in the case of polythiol compounds, also can be avoided completely. In addition, the use of a polythiol's lithium salt instead of the polythiol itself has solved the problem of thiol vaporization in the process of drying the positive electrode because replacing a liquid polythiol with its salt which is solid makes the pertinent boiling point much higher; the use of a polythiol's lithium salt has made broader the range of polythiols usable to this application.
Further the inventors have found that the isolation of lithium thiolates and elimination of water from them by drying improve the cycle characteristics so much that even the use of metallic lithium as a negative electrode can give a good result. Take DMc T for example. DMc T is known to decompose at its melting point, i. e., at about 170°C but the decomposition temperature decreases when impurities are contained; according to the inventors' study, the decomposition temperature is lowered to about 140°C when the impurities from oxidation of DMc T are contained by several percent; thus DM c T cannot be said to be advantageous as a material for batteries. In contrast, DMc T dilithium salt represented by the formula (1) above has proved to have heat-stability improved remarkably, having a decomposition temperature of about 340°C. DMc T-2 L i is usually produced in the form of dihydrate, but it has been found that DM c T-2 L i can be made anhydrous by drying at 200°C.
In the past the use of metallic lithium as a negative electrode material posed problems such as hydrogen gas generation during the initial charging and discharging, and the cycle characteristics lowering, but the inventors have solved these problems by once isolating DM c T-2 L i dihydrate and then drying it enough to make it anhydrous. As described hitherto, the inventors' study has found positive electrode-active materials for batteries which can improve cycle characteristics, prevent hydrogen gas generation and provide safety, and achive high energy density; thus this invention has been completed.
The thermal analysis of DMc T-2 L i dihydrate, the new compound discoverd in this invention, has shown that it has a decomposition temperature of about 341°C and has much higher heat-stability than DMc T. As this invention shows, DMc T-2 L i dihydrate of such a high quality is obtained when the mole ration of lithium to DMc T in the reaction to produce the dilithium salt is adjusted to 2. DMc T-2 L i dihydrate is more soluble in polar solvents such as water, alcohol, etc. than DM c T, so it can improve the volume efficiency. It also brings another remarkable advantage: its high heat-stability enables it to be applied to areas for which DM c T cannot be used.

### [Examples]

The invention is illustrated in the following examples but is not limited only to the examples.

### [Examples 1]

0.63 g (14.90 mmol) of lithium hydroxide monohydrate (content 99.95%) and 5.9 g of distilled water were charged to a reaction vessel and the lithium hydroxide monohydrate was dissolved. 150 ml of ethanol was added to this solution. The mixture was made homogeneous by agitation and then the atmosphere was replaced with nitrogen. 275 ml of ethanol and 275 ml of diethyl ether were placed in another vessel, whose atmosphere was then replaced with nitrogen. To this vessel 1.12 g (7.45mmol) of DMcT (content 99%) was charged and replacement with nitrogen was conducted again. After that, the alcoholic solution of lithium hydroxide already prepared was added dropwise to the DM c T solution, while the temperature of the droplets of the lithium hydroxide solution was kept at 20°C or lower. After completion of the dropwise addition, the mixture was stirred for 30 minutes at a reaction temperature of 20°C, and then the mixture was subjected to concentration under reduced pressure to distill out ethanol and diethyl ether. To the remaining solution 300 ml of toluene was added and concentration under reduced pressure was applied again.After repeating this procedure three times, 10 ml of toluene was added to the remaining solution; then the crystal matter was separated by filtration and was dried under vacuum at 60°C for 24 hours. Thus 1.47 g of DM c T dilithium salt dihydrate, a white crystal, was obtained(yield 99%). The results of elementary analysis, thermal analysis, and NMR are shown below;

| | | |
|---|---|---|
| ¹H | NMR δₚₚₘ | 4.74 (s. H₂O) |
| ¹³C | NMR δₚₚₘ | 177.48 (s. C) |

| Elementary analysis | | | | |
|---|---|---|---|---|
| | C | H | N | S |
| Observed values | 12.01 | 2.27 | 14.09 | 48.51 |
| Calculated values | 12.12 | 2.04 | 14.14 | 48.55 |

| Thermal analysis | |
|---|---|
| Weight loss beginning point | 183.1°C (weight loss 18.2%) |
| | 329.6°C |
| Endothermic peak | 194.2°C |
| Exothermic peak | 341.6°C |

### [Example 2]

1.00 g of DMc T dilithium salt dithydrate obtained by the method of Example 1 was dried for 24 hours at 200°C under reduced pressure. Thus 0.81 g of DMc T dilithium salt, a white crystal, was obtained (yield 99%). The results of NM R spectroscopy analysis and elementary analysis are shown below:

| NMR spectroscopy analysis | | |
|---|---|---|
| ¹H | NMR δ ₚₚₘ | peak is not detected |
| ¹³C | NMR δ ₚₚₘ | 177.82 (s . C) |

| Elementary analysis | | | | |
|---|---|---|---|---|
| | C | H | N | S |
| Observed values | 14.71 | 0.03 | 17.21 | 59.16 |
| Calculated values | 14.81 | 0.00 | 17.28 | 59.34 |

### [Reference Example 3]

0.1 g (14.90 mmol) of Metalic Li (purity 99.9%) and 100 ml of ethanol charged to a reaction vessel and then the atmosphere was replaced with nitrogen. 275 ml of ethanol and 275 ml of diethylether were placed in another vessel, Whose atmosphere was then replaced with nitrogen. Previous prepared alcoholic solution of metalic Li is poured with that vessel under the temperature of 20°C.
After the above pouring of that alcoholic solution into the vessel is finished, said alcoholic solution is agitated for 30 minites,at 20°C and ethanol and diethyler
are removed by distillation under redueed pressure and then 10 ml toluene is added to the remained alcoholic solution.
And after the solution is agitatd,obtained crystals are filtered and dried at 60°C for 18 hours under reduced pressere.
Thus 1.2 g of DMc T dilithum salt, a white crystal,was obtained(yield 99%).

### [Reference Example 4]

1.5 weight parts of anhydrous DM c T dilithium salt was dissolved in 7.0 weight parts of NMP and a homogeneous viscous solution was obtained. To this solution 1.0 weight part of polyaniline was added and dissolved to produce a homogeneous solution. To this solution 5 weight parts of acetylene black was added and dispersed uniformly by agitation.This solution was applied to a piece of 30-µm-thick Cu foil with 1 µm Ag plating, and the foil piece was dried under vacuum at 80°C for one hours. Thus a positive electrode of a 20 mmx 20 mm sheet was obtained. As a negative electrode, a 300- µm- thick,20 mmx 20 mm lithium foil piece was used.Lithium borofluride was added to a 1:1 mixed solution of propylene carbonate and ethylene carbonate; to the resultant solution a copolymer of acrylonitrile and vinyl acetate was added, and the mixture was stirred to make a homogeneous solution; then by cooling the solution to 20°C a polymer gel electrolyte was obtained. The composite positve electrode, negative electrode, and electrolyte thus obtained, were put in a sealed alminum laminate container; this formed a battery.

### [Effects of the Invention]

DMc T dilithium salt and its dihydrate of invention are more soluble in polar solvents and much more heat-stable than DMc T, so they can be used not only in the application areas in wich DMc T has been used but also in areas for wich DMc T has not been suitable. This is a remarkable advantage of theirs. Furthermore, the dilithium salt of this invention are positive electrodeactive materials for batteries which will provide good cycle characteristics and good safety, and also are compounds which will enable the development of what is sought today, i.e., a secondary battery having high energy density. Further, it has been confirmed that the use of the anhydrous lithium thiolate of invention enables the manufacture of good batteries even when metalli clithium is used as a negative electrode material. Besides the use of the anhydrous lithium thiolate of this invention enables the manufacture of secondary batteries having no positive electrode film expansion,which expansion has been a problem in the use of thiol compounds; thus the thiolate is a material which can be used in commercial battery production.

### [Brief Explanation of the Drawings]

### [Figure 1]

The chart of the infrared absorption spectrum of the compound of Example 1 in this invention is shown below:

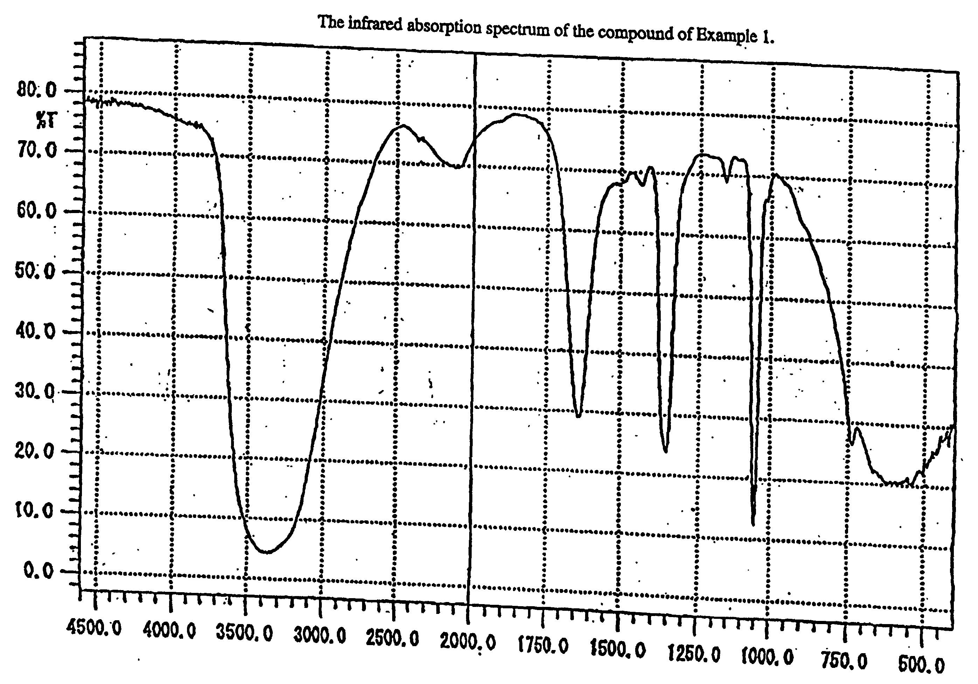

## Claims

1. A dilithium salt dihydrate of 2,5-dimercapto-1,3,4-thiadiazole (abbreviated to DMc T hereafter), represented by the formula (1):

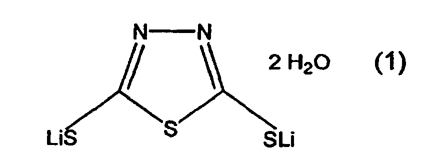

2. A method for the manufacture of DMc T dilithium salt dihydrate of formula (1) as defined in claim 1 in which DMc T is allowed to react with a lithiation agent in a solvent at 0-120°C, the mole ratio of lithium in the lithiation agent to DMc T being adjusted to 1.8-3.0.

3. A method for the manufacture of DMc T dilithium salt represented by the formula (2), which method comprises heating and dehydrating DMc T dilithium salt dihydrate represented by the formula (1) at 200°C or above and drying the product.

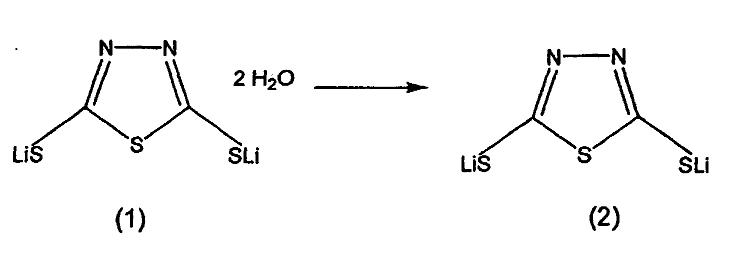

## Patentansprüche

1. Dilithiumsalzdihydrat von 2,5-Dimercapto-1,3,4-thiadiazol (abgekürzt als DMc T) mit der Formel (1)

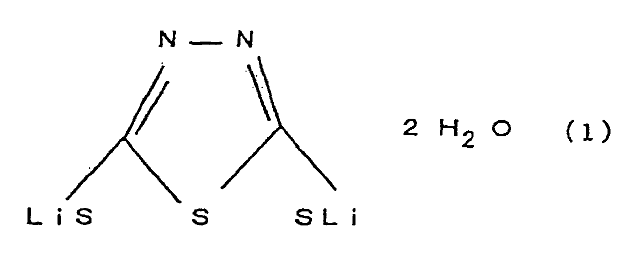

2. Verfahren zur Herstellung von DMc T-Dilithiumsalzdihydrat der Formel (1) wie in Anspruch 1 definiert, bei dem DMc T mit einem Lithiierungsmittel in einem Lösungsmittel bei 0 bis 120°C reagieren gelassen wird, wobei das Molverhältnis von Lithium in dem Lithiierungsmittel zu DMc T auf 1,8 bis 3,0 eingestellt wird.

3. Verfahren zur Herstellung von DMc T-Dilithiumsalz der Formel (2), wobei das Verfahren beinhaltet, dass DMc T-Dilithiumsalzdihydrat der Formel (1) bei 200°C oder mehr erwärmt und dehydratisiert wird und das Produkt getrocknet wird.

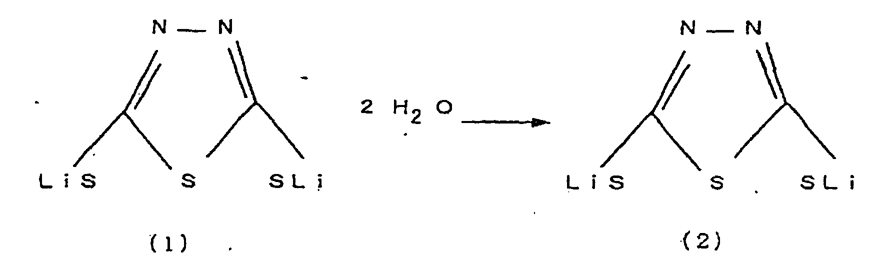

## Revendications

1. Sel de dilithium du 2,5-dimercapto-1,3,4-thiadiazole (abrégé DMc T ci-après) dihydraté, de formule (1) :

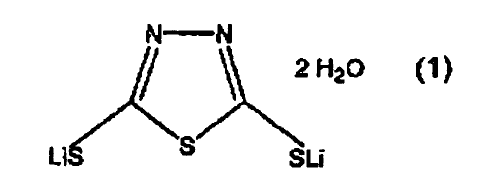

2. Procédé de fabrication du sel de dilithium du DMc T dihydraté de formule (1) comme défini selon la revendication 1 dans lequel le DMc T est laissé réagir avec un agent de lithiation dans un solvant à une température de 0 à 120°C, le rapport molaire du lithium dans l'agent de lithiation sur le DMc T étant ajusté à une valeur de 1,8 à 3,0.

3. Procédé de fabrication du sel de dilithium du DMc T de formule (2), le procédé comprenant les étapes consistant à chauffer et à déshydrater le sel de dilithium du DMc T dihydraté de formule (1) à 200°C ou plus et à sécher le produit.
